# EUROPEAN PATENT APPLICATION

(11) **EP 2 865 338 A1**
(43) Date of publication of application: **29.04.2015**
(21) Application number: 14185517.1
(22) Date of filing: 19.09.2014
(51) Int. Cl.: A61B 8/00, G01S 7/52

(54) **Ultrasound diagnosis apparatus and time gain compensation (TGC) setting method performed by the ultrasound diagnosis apparatus**

(30) Priority: 24.10.2013 KR 20130127304; 14.05.2014 KR 20140057945
(71) Applicant: Samsung Medison Co., Ltd., Gangwon-do 250-870 (KR)
(72) Inventor: Yoon, Ki-sang, Gangwon-do (KR)
(74) Representative: Frey, Sven Holger

(57) **Abstract**

An ultrasound diagnosis apparatus (100) includes a display unit (25) which displays a user interface (UI) image for setting a plurality of time gain compensation (TGC) values according to depths; a UI unit (50) which receives a first input for setting a TGC value corresponding to a first depth from among the plurality of TGC values to be a first TGC value, via the UI image; and a control unit (60) which sets the TGC value corresponding to the first depth to be the first TGC value, based on the first input, and automatically sets a TGC value corresponding to at least one second depth, except for the TGC value corresponding to the first depth, from among the plurality of TGC values, based on the first TGC value.

## Description

### RELATED APPLICATIONS

This application claims the benefits of Korean Patent Application No. 10-2013-0127304, filed on October 24, 2013, and Korean Patent Application No. 10-2014-0057945, filed on May 14, 2014, in the Korean Intellectual Property Office, the disclosures of which are incorporated herein in its entirety by reference.

### BACKGROUND

### 1. Field

One or more embodiments of the present invention relate to an ultrasound diagnosis apparatus capable of efficiently setting time gain compensation (TGC).

### 2. Description of the Related Art

Ultrasound diagnosis apparatuses radiate an ultrasound signal generated by a transducer of a probe toward an object and receive information regarding an ultrasound echo signal reflected from the object, thereby obtaining an image of an internal part of the object. In particular, ultrasound diagnosis apparatuses are used for medical purposes, such as observation of the inside of an object, detection of foreign substances inside the object, and diagnosis of damage thereof. Such ultrasound diagnosis apparatuses have various advantages, including stability, real-time display, and safety because of no radioactive exposure, compared to X-ray apparatuses, and thus, the ultrasound diagnosis apparatuses are commonly used together with other image diagnosis apparatuses.

In general, an ultrasound echo signal reflected from an object after passing through the tissue of the inside of an object has an amplitude or intensity attenuated according to distances by which the ultrasound echo signal has passed. As an ultrasound echo signal passes longer distances, the amplitude thereof is attenuated more. Accordingly, an ultrasound echo signal reflected from a part of an object that is deep from the surface of a probe has smaller amplitude than that reflected from a part of the object that is close to the surface of the probe. Due to the attenuation of the amplitude of an ultrasound echo signal according to distances or depths, the brightness of an ultrasound image may be non-uniform, or the quality thereof may be partially degraded. In detail, due to the attenuation of the amplitude of an ultrasound echo signal, a part of an object that is deep from the surface of a probe appears darker than a part of the object that is close to the surface of the probe.

To compensate for the attenuation of an ultrasound echo signal, different gains are applied to ultrasound echo signals that are collected from different parts of an object. For example, the gains are time gains, and compensation of an ultrasound echo signal by using time gains may be time gain compensation (TGC). Hereinafter, a TGC value that is applied to an ultrasound echo signal is referred to as a TGC value.

When an ultrasound image is acquired, the quality thereof may vary according to how TGC values are set. Accordingly, there is a demand for an ultrasound diagnosis apparatus and method capable of easily and conveniently performing TGC.

### SUMMARY

One or more embodiments of the present invention include an ultrasound diagnosis apparatus that allows a user to easily and conveniently set time gain compensation (TGC) values, and a TGC setting method performed by the ultrasound diagnosis apparatus.

Additional aspects will be set forth in part in the description which follows and, in part, will be apparent from the description, or may be learned by practice of the presented embodiments.

According to one or more embodiments of the present invention, an ultrasound diagnosis apparatus includes a display unit which displays a user interface (UI) image for setting a plurality of TGC values according to depths; a UI unit which receives a first input for setting a TGC value corresponding to a first depth from among the plurality of TGC values to be a first TGC value, via the UI image; and a control unit which sets the TGC value corresponding to the first depth to be the first TGC value, based on the first input, and automatically sets a TGC value corresponding to at least one second depth except for the TGC value corresponding to the first depth from among the plurality of TGC values, based on the first TGC value.

The control unit may set the TGC value corresponding to the at least one second depth to be greater or smaller than the first TGC value corresponding to the first depth as the at least one second depth increases or decreases from the first depth.

The control unit may set the TGC value corresponding to the at least one second depth to be smaller than the first TGC value corresponding to the first depth when the at least one second depth decreases from the first depth.

The control unit may set the TGC value corresponding to the at least one second depth to be smaller than the first TGC value corresponding to the first depth when the at least one second depth increases from the first depth.

The control unit may set the TGC value corresponding to the at least one second depth to be greater than the first TGC value corresponding to the first depth when the at least one second depth increases from the first depth.

The control unit may set the TGC value corresponding to the at least one second depth to form a straight line pattern together with the first TGC value corresponding to the first depth.

The control unit may set the TGC value corresponding to the at least one second depth to form a curved line pattern together with the first TGC value corresponding to the first depth.

The control unit may set the TGC value corresponding to the at least one second depth to form a user-set pattern together with the first TGC value corresponding to the first depth.

The control unit may set the TGC value corresponding to the at least one second depth to be the same as the first TGC value.

The control unit may set the TGC value corresponding to the at least one second depth, based on a part of an object that is to be ultrasound-diagnosed.

According to one or more embodiments of the present invention, an ultrasound diagnosis apparatus includes a display unit which displays a UI image for setting a plurality of TGC values according to depths; a UI unit which receives a first input and a second input for respectively setting a TGC value corresponding to a first depth and a TGC value corresponding to a second depth from among the plurality of TGC values to be first and second TGC values, respectively, via the UI image; and a control unit which sets the TGC values corresponding to the first and second depths as the first and second TGC values, respectively, based on the first and second inputs, and automatically sets a TGC value corresponding to at least one third depth, except for the TGC values corresponding to the first and second depths, based on the first and second TGC values.

According to one or more embodiments of the present invention, a TGC setting method performed by an ultrasound diagnosis apparatus includes displaying a UI image for setting a plurality of TGC values according to depths; receiving a first input for setting a TGC value corresponding to a first depth from among the plurality of TGC values to be a first TGC value, via the UI image; and setting the TGC value corresponding to the first depth to be the first TGC value, based on the first input and automatically setting a TGC value corresponding to at least one second depth, except for the TGC value corresponding to the first depth, from among the plurality of TGC values, based on the first TGC value.

The automatically setting of the TGC value corresponding to the at least one second depth may include setting the TGC value corresponding to the at least one second depth to be greater or smaller than the first TGC value corresponding to the first depth as the at least one second depth increases or decreases from the first depth.

The automatically setting of the TGC value corresponding to the at least one second depth may include setting the TGC value corresponding to the at least one second depth to be smaller than the first TGC value corresponding to the first depth when the at least one second depth decreases from the first depth.

The automatically setting of the TGC value corresponding to the at least one second depth may include setting the TGC value corresponding to the at least one second depth to be smaller than the first TGC value corresponding to the first depth when the at least one second depth increases from the first depth.

The automatically setting of the TGC value corresponding to the at least one second depth may include setting the TGC value corresponding to the at least one second depth to be greater than the first TGC value corresponding to the first depth when the at least one second depth increases from the first depth.

The automatically setting of the TGC value corresponding to the at least one second depth may include setting the TGC value corresponding to the at least one second depth to form a straight line pattern together with the first TGC value corresponding to the first depth.

The automatically setting of the TGC value corresponding to the at least one second depth may include setting the TGC value corresponding to the at least one second depth to form a curved line pattern together with the first TGC value corresponding to the first depth.

The automatically setting of the TGC value corresponding to the at least one second depth may include setting the TGC value corresponding to the at least one second depth to form a user-set pattern together with the first TGC value corresponding to the first depth.

The automatically setting of the TGC value corresponding to the at least one second depth may include setting the TGC value corresponding to the at least one second depth to be the same as the first TGC value.

The automatically setting of the TGC value corresponding to the at least one second depth may include setting the TGC value corresponding to the at least one second depth, based on a part of an object that is to be ultrasound-diagnosed.

According to one or more embodiments of the present invention, aTGC setting method performed by an ultrasound diagnosis apparatus includes displaying a UI image for setting a plurality of TGC values according to depths; receiving a first input and a second input for respectively setting a TGC value corresponding to a first depth and a TGC value corresponding to a second depth from among the plurality of TGC values to be first and second TGC values, respectively, via the UI image; and setting the TGC values corresponding to the first and second depths as the first and second TGC values, respectively, based on the first and second inputs, and automatically setting a TGC value corresponding to at least one third depth, except for the TGC values corresponding to the first and second depths, based on the first and second TGC values.

According to one or more embodiments of the present invention, a computer-readable recording medium has recorded thereon a program for executing the above-described methods.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram of an ultrasound diagnosis apparatus according to an embodiment of the present invention;
FIG. 2 is a block diagram of a wireless probe according to an embodiment of the present invention;
FIG. 3 is a block diagram of an ultrasound diagnosis apparatus according to an embodiment of the present invention;
FIG. 4 is a block diagram of an ultrasound diagnosis apparatus according to another embodiment of the present invention;
FIGS. 5A, 5B, and 5C illustrate embodiments of the present invention in which a user input is received;
FIG. 6 is a view for describing a general operation of an ultrasound diagnosis apparatus to set time gain compensation (TGC) values;
FIG. 7 is a view for describing an operation of an ultrasound diagnosis apparatus to set TGC values, according to an embodiment of the present invention;
FIG. 8 is a view for describing an operation of an ultrasound diagnosis apparatus to set TGC values, according to another embodiment of the present invention;
FIG. 9 is a view for describing an operation of an ultrasound diagnosis apparatus to set TGC values, according to another embodiment of the present invention;
FIG. 10 is a view for describing an operation of an ultrasound diagnosis apparatus to set TGC values, according to another embodiment of the present invention;
FIG. 11 is a view for describing an operation of an ultrasound diagnosis apparatus to set TGC values, according to another embodiment of the present invention;
FIG. 12 is a view for describing an operation of an ultrasound diagnosis apparatus to set TGC values, according to another embodiment of the present invention;
FIG. 13 is a view for describing an operation of an ultrasound diagnosis apparatus to set TGC values, according to another embodiment of the present invention;
FIG. 14 is a view for describing an operation of an ultrasound diagnosis apparatus to set TGC values, according to another embodiment of the present invention;
FIG. 15 is a view for describing an operation of an ultrasound diagnosis apparatus to set TGC values, according to another embodiment of the present invention;
FIG. 16 is a view for describing an operation of an ultrasound diagnosis apparatus to set TGC values, according to another embodiment of the present invention;
FIG. 17 is a flowchart of a method in which an ultrasound diagnosis apparatus sets TGC values, according to an embodiment of the present invention; and
FIG. 18 is a flowchart of a method in which an ultrasound diagnosis apparatus sets a TGC value, according to another embodiment of the present invention.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

Although general terms widely used at present were selected for describing the present invention in consideration of the functions thereof, these general terms may vary according to intentions of one of ordinary skill in the art, case precedents, the advent of new technologies, and the like. Terms arbitrarily selected by the applicant of the present invention may also be used in a specific case. In this case, their meanings need to be given in the detailed description of the present invention. Hence, the terms must be defined based on their meanings and the contents of the entire specification, not by simply stating the terms.

The terms "comprises" and/or "comprising" or "includes" and/or "including" when used in this specification, specify the presence of stated elements, but do not preclude the presence or addition of one or more other elements. In addition, terms such as "... unit", "... module", or the like refer to units that perform at least one function or operation, and the units may be implemented as hardware or software or as a combination of hardware and software.

Throughout the specification, an "ultrasound image" refers to an image of an object obtained using ultrasound waves. Throughout the specification, "object" may include a person, animal, or a part of a person or animal. For example, the object may include the liver, the heart, the womb, the brain, a breast, the abdomen, or a blood vessel. The object may include a phantom. The phantom means a material having a volume that is approximately the intensity and effective atomic number of a living thing.

Throughout the specification, "user" refers to a medical professional, such as a doctor, a nurse, a medical laboratory technologist, and an engineer who repairs a medical apparatus, but the user is not limited thereto.

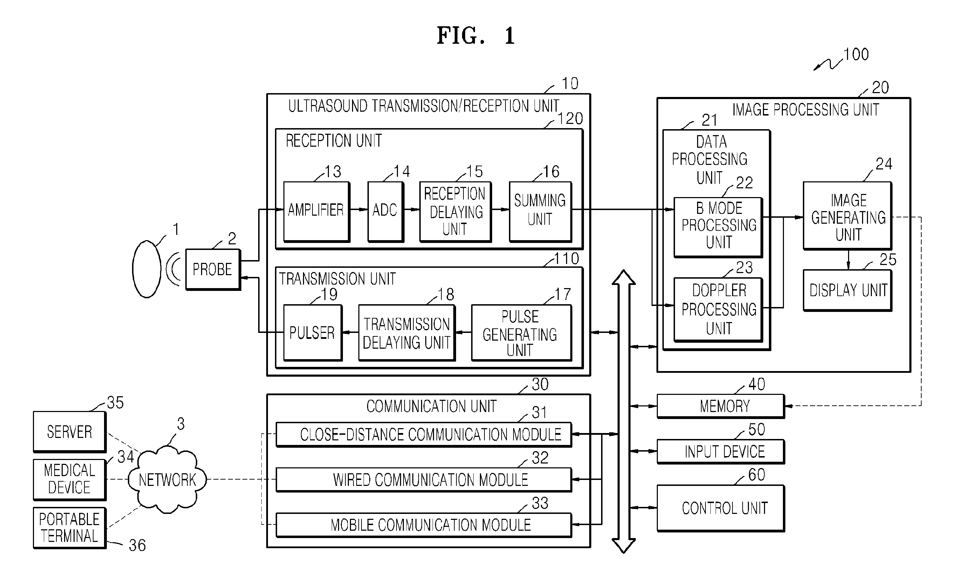

FIG. 1 is a block diagram of an ultrasound diagnosis apparatus 100 according to an embodiment of the present invention.

Referring to FIG. 1, a structure of the ultrasound diagnosis apparatus 100 is illustrated.

Referring to FIG. 1, the ultrasound diagnosis apparatus 100 may include a probe 2, an ultrasound transmission/reception unit 10, an image processing unit 20, a communication unit 30, a memory 40, an input device 50, and a control unit 60, which may be connected to one another via buses 70.

The ultrasound diagnosis apparatus 100 may be not only a cart-type apparatus, but also a portable apparatus. Examples of portable ultrasound diagnosis apparatuses may include, but are not limited thereto, a picture archiving and communication system (PACS) viewer, a smartphone, a laptop computer, a personal digital assistant (PDA), and a tablet PC.

The probe 2 transmits ultrasound waves to an object 1 based on a driving signal applied by the ultrasound transmission/reception unit 10 and receives echo signals reflected by the object 1. The probe 2 includes a plurality of transducers, and the plurality of transducers oscillate based on electric signals transmitted thereto and generate acoustic energy, that is, ultrasound waves. Furthermore, the probe 2 may be connected to the main body of the ultrasound diagnosis apparatus 100 by wires or wirelessly. According to embodiments of the present invention, the ultrasound diagnosis apparatus 100 may include a plurality of probes 2.

A transmission unit 110 supplies a driving signal to the probe 2. The transmission unit 110 includes a pulse generating unit 17, a transmission delaying unit 18, and a pulser 19. The pulse generating unit 17 generates pulses for forming transmission ultrasound waves based on a predetermined pulse repetition frequency (PRF), and the transmission delaying unit 18 applies a delay time necessary for determining transmission directionality to the pulses. Pulses, to which a delay time has been applied, correspond to a plurality of piezoelectric vibrators included in the probe 2, respectively. The pulser 19 applies a driving signal or a driving pulse to the probe 2 based on timing that corresponds to each of the pulses having the delay time applied thereto.

A reception unit 120 generates ultrasound data by processing ultrasound echo signals received from the probe 2. The reception unit 120 may include an amplifier 13, an analog-digital converter (ADC) 14, a reception delaying unit 15, and a summing unit 16. The amplifier 13 amplifies the ultrasound echo signals in each channel, and the ADC 14 performs analog-to-digital conversion on the amplified ultrasound echo signals. The reception delaying unit 15 applies delay times necessary for determining reception directionality to digital ultrasound echo signals obtained by the analog-to-digital conversion, and the summing unit 16 generates ultrasound data by summing the ultrasound echo signals processed by the reception delaying unit 15. According to embodiments, the reception unit 120 may not include the amplifier 13. In other words, if the sensitivity of the probe 2 or the capability to process bits by the ADC 14 are enhanced, the amplifier 13 may be omitted.

The image processing unit 20 generates an ultrasound image by scan-converting ultrasound data generated by the ultrasound transmission/reception unit 10 and displays the ultrasound image. An ultrasound image may include not only a grayscale ultrasound image obtained by scanning an object in an amplitude (A) mode, a brightness (B) mode, and a motion (M) mode, but also a Doppler image showing a movement of an object via a Doppler effect. The Doppler image may be a blood flow Doppler image showing flow of blood (or a color Doppler image), a tissue Doppler image showing a movement of tissue, or a spectral Doppler image showing a moving speed of an object as a waveform.

A B mode processing unit 22 extracts B mode components from ultrasound data and processes the B mode components. An image generating unit 24 may generate an ultrasound image indicating signal intensities as brightness based on the extracted B mode components.

Similarly, a Doppler processing unit 23 may extract Doppler components from ultrasound data, and the image generating unit 24 may generate a Doppler image indicating a movement of an object as colors or waveforms based on the extracted Doppler components.

According to an embodiment, the image generating unit 24 may generate a 3D ultrasound image via volume rendering with respect to volume data and may also generate an elasticity image by imaging deformation of the object 1 due to pressure. Furthermore, the image generating unit 24 may display various pieces of additional information in an ultrasound image by using text and graphics. In addition, the generated ultrasound image may be stored in the memory 40.

A display unit 25 displays the generated ultrasound image. The display unit 25 may display not only an ultrasound image, but also various pieces of information processed by the ultrasound diagnosis apparatus 100 on a screen image via a graphical user interface (GUI). In addition, the ultrasound diagnosis apparatus 100 may include two or more display units 25 according to embodiments of the present invention.

The communication unit 30 is connected to a network 3 in a wired or wireless manner to communicate with an external device or server. The communication unit 30 may exchange data with a hospital server or other medical apparatuses in a hospital that is or are connected thereto via a medical image information system (e.g., a PACS). The communication unit 30 may perform data communication according to the Digital Imaging and Communications in Medicine (DICOM) Standard.

In detail, the communication unit 30 may transmit or receive data related to the diagnosis of an object, such as an ultrasound image, ultrasound data, Doppler data, etc. of the object 1, through the network 3, and may also transmit or receive a medical image captured by another medical apparatus, such as a CT apparatus, an MRI apparatus, or an X-ray apparatus. Furthermore, the communication unit 30 may receive information about a diagnosis history or a medical treatment schedule of a patient from a server and utilize the received information to diagnose the object 1. In addition, the communication unit 30 may perform data communication with a portable terminal of a medical doctor or a patient, in addition to a server or medical apparatus of a hospital.

The communication unit 30 may be by wires or wirelessly connected to the network 3 and thus may exchange data with a server 35, a medical apparatus 34, or a portable terminal 36. The communication unit 30 may include one or more elements for communication with an external device. For example, the communication unit 30 may include a close-distance communication module 31, a wired communication module 32, and a mobile communication module 33.

The close-distance communication module 31 refers to a module for close-distance communication within a predetermined distance. Examples of close-distance communication techniques according to an embodiment of the present invention may include, but are not limited thereto, wireless LAN, Wi-Fi, Bluetooth, ZigBee, Wi-Fi direct (WFD), ultra wideband (UWB), infrared data association (IrDA), Bluetooth low energy (BLE), and near field communication (NFC).

The wired communication module 32 refers to a module for communication using electric signals or optical signals. Examples of wired communication techniques according to an embodiment of the present invention may include a pair cable, a coaxial cable, an optical fiber cable, and an Ethernet cable.

The mobile communication module 33 transmits or receives wireless signals with at least one from among a base station, an external terminal, and a server on a mobile communication network. Here, the wireless signals may include voice call signals, video call signals, or various types of data for transmission and reception of text/multimedia messages.

The memory 40 stores various data processed by the ultrasound diagnosis apparatus 100. For example, the memory 40 may store medical data related to diagnosis of an object, such as ultrasound data and an ultrasound image that are input or output, and may also store algorithms or programs which are to be executed in the ultrasound diagnosis apparatus 100.

The memory 400 may be any of various storage media, e.g., a flash memory, a hard disk drive, EEPROM, etc. The ultrasound diagnosis apparatus 100 may utilize a web storage or a cloud server which performs a storage function of the memory 40 on the web.

The input device 50 denotes a unit via which a user inputs data for controlling the ultrasound diagnosis apparatus 100. The input device 50 may include hardware components, such as a keypad, a mouse, a touch pad, a touch screen, and a jog switch. However, embodiments of the present invention are not limited thereto, and the input device 50 may further include various other input means including an electrocardiogram measuring module, a respiration measuring module, a voice recognition sensor, a gesture recognition sensor, a fingerprint recognition sensor, an iris recognition sensor, a depth sensor, a distance sensor, etc.

The control unit 60 controls all operations of the ultrasound diagnosis apparatus 100. In other words, the control unit 60 may control operations among the probe 2, the ultrasound transmission/reception unit 10, the image processing unit 20, the communication unit 30, the memory 40, and the input device 50 shown in FIG. 1.

All or some of the probe 2, the ultrasound transmission/reception unit 10, the image processing unit 20, the communication unit 30, the memory 40, the input device 50, and the control unit 60 may be operated by software modules. However, embodiments of the present invention are not limited thereto, and some of the components stated above may be operated by hardware modules. Furthermore, at least some of the ultrasound transmission/reception unit 10, the image processing unit 20, and the communication unit 30 may be included in the control unit 60. However, embodiments of the present invention are not limited thereto.

To diagnose a disease by using an ultrasound image, a marker may be set to set a diagnosis part within the ultrasound image including an object or to indicate a predetermined location therewithin.

In detail, the marker may be set on a part of the object that a user requires to carefully observe to diagnose a disease or detect whether a patient is healthy. In order to more accurately diagnose a part of an object on which the above-described marker was set, the present application is directed to an ultrasound diagnosis apparatus that changes an ultrasound image and outputs a changed ultrasound image, and a method of displaying an ultrasound image by the ultrasound diagnosis apparatus.

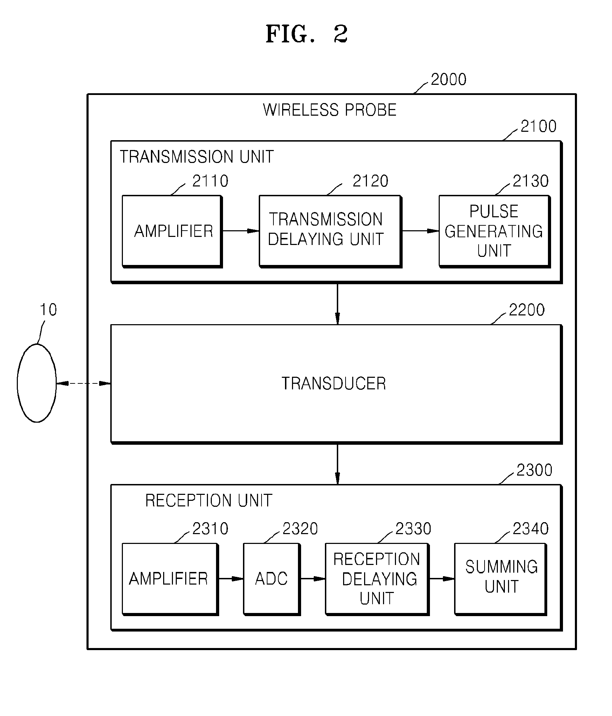

FIG. 2 is a block diagram of a wireless probe 2000 according to an embodiment of the present invention. As described above with reference to FIG. 1, the wireless probe 2000 may include a plurality of transducers, and, according to embodiments of the present invention, may include some or all of the components of the ultrasound transmission/reception unit 10 shown in FIG. 1.

The wireless probe 2000 according to the embodiment shown in FIG. 2 includes a transmission unit 2100, a transducer 2200, and a reception unit 2300. Since descriptions thereof are given above with reference to FIG. 1, detailed descriptions thereof will be omitted. In addition, according to embodiments of the present invention, the wireless probe 2000 may selectively include a reception delaying unit 2330 and a summing unit 2340.

The wireless probe 2000 may transmit ultrasound signals to an object 1, receive ultrasound echo signals from the object 10, generate ultrasound data, and transmit the ultrasound data to the ultrasound diagnosis apparatus 100 of FIG. 1 wirelessly.

As described above, the magnitudes of ultrasound echo signals that an ultrasound diagnosis apparatus has received are not consistent, because several factors, such as, a transmission distance, a reflection coefficient, a medium, and a temperature, affect the magnitudes of the ultrasound echo signals. Accordingly, when the magnitudes of the ultrasound echo signals are compensated for considering all of these factors, an accurate ultrasound image of an object may be obtained.

Compensating for the magnitudes of ultrasound echo signals according to transmission distances of the ultrasound echo signals or depths at which parts of an object are located is referred to as time gain compensation (TGC) or depth gain compensation (DGC).

In other words, TGC is a method of compensating for a difference between the magnitudes of ultrasound echo signals received from reflectors which are located at different depths. In detail, assuming that ultrasound echo signals have the same speed, as a reflector is locateddeeper, a transmission distance of an ultrasound echo signal reflected from the reflector is longer, an arrival time thereof gets longer, and attenuation thereof increases. Accordingly, as an ultrasound echo signal arrives later, greater compensation occurs. TGC denotes converting different depths of reflectors into time and compensating for the time and has the same meaning as DGC.

In general, to perform TGC, different TGC values need to be set according to different depths or distances. Thus, a user is incovenienced in having to individually set a TGC value for each of a plurality of depths. Therefore, a method of efficiently and conveniently setting a plurality of TGC values is needed.

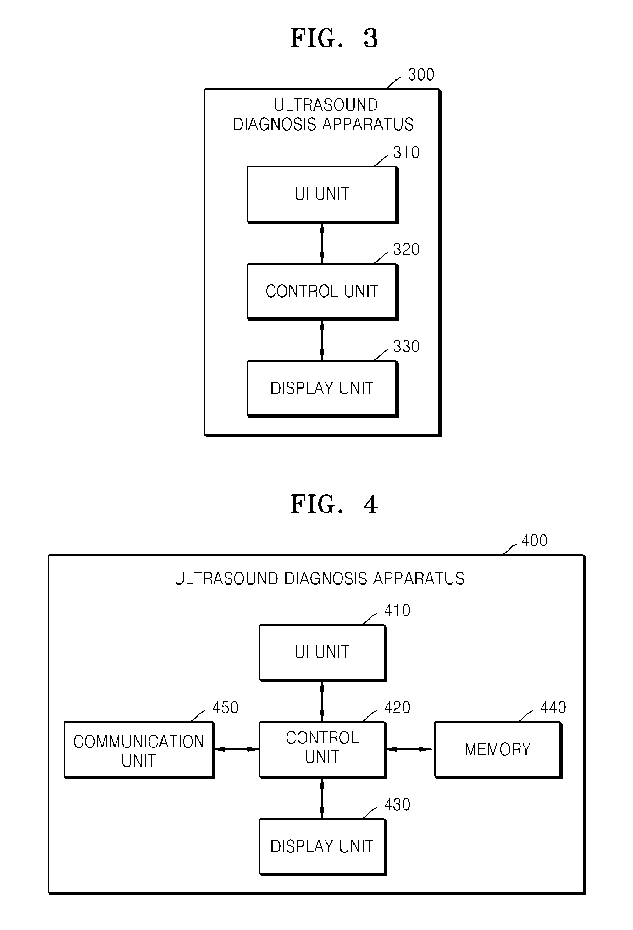

An ultrasound diagnosis apparatus according to one or more embodiments of the present invention will now be described in detail with reference to FIGS. 3-18.

The ultrasound diagnosis apparatus according to one or more embodiments of the present invention may be included in the ultrasound diagnosis apparatus 100 described above with reference to FIGS. 1 and 2. Alternatively, the ultrasound diagnosis apparatus according to one or more embodiments of the present invention may be included in the medical apparatus 34 or the portable terminal 36 connected to the ultrasound diagnosis apparatus 100 via the network 3.

FIG. 3 is a block diagram of an ultrasound diagnosis apparatus 300 according to an embodiment of the present invention.

Referring to FIG. 3, the ultrasound diagnosis apparatus 300 includes a UI unit 310, a control unit 320, and a display unit 330.

When the ultrasound diagnosis apparatus 300 is included in the ultrasound diagnosis apparatus 100 of FIG. 1, the UI unit 310, the control unit 320, and the display unit 330 of FIG. 3 may respectively correspond to the input device 50, the control unit 60, and the display unit 25 of FIG. 1. Accordingly, descriptions of the ultrasound diagnosis apparatus 300 that are the same as those made with reference to FIG. 1 are not repeated herein.

Alternatively, the ultrasound diagnosis apparatus 300 may be included in the medical apparatus 34 or the portable terminal 36 of FIG. 1.

The display unit 330 displays a UI image for setting a plurality of TGC values according to depths. The horizontal axis of the UI image may indicate a brightness value that denotes TGC, and the vertical axis thereof may indicate a depth. A user may set a plurality of TGC values for different depths, respectively.

The UI unit 310 receives an input via the UI image displayed on the display unit 330.

The UI unit 310 receives a predetermined request, a predetermined command, or other data from the user. In detail, the UI unit 310 may receive a first input for setting, as a first TGC value, a TGC value that corresponds to a first depth and that a user desires to be set from among the plurality of TGC values. The UI unit 310 may also receive a second input for setting a TGC value corresponding to a second depth from among the plurality of TGC values to be a second TGC value. A user input and an operation of an ultrasound diagnosis apparatus in response to the user input will be described in detail with reference to FIGS. 3-18.

The UI unit 310 may be a touch pad. In detail, the UI unit 310 may include a touch pad (not shown) coupled with a display panel (not shown) included in the display unit 330. The display unit 330 displays the UI image on the display panel. When a user inputs a command by touching a certain point on the UI image, the touch pad may sense the input operation and recognize the command input by the user.

In detail, when the UI unit 310 is a touch pad and the user touches a certain point on the UI image, the UI unit 310 senses the touched point. Then, the UI unit 310 may transmit sensed information to the control unit 320. Thereafter, the control unit 320 may recognize a user's request or command corresponding to the sensed information and may perform the recognized user's request or command.

The control unit 320, the display unit 330, and the UI unit 310 may be connected to each other via wires or wirelessly and may transmit or receive data to or from each other.

The control unit 320 sets the TGC value corresponding to the first depth to be the first TGC value, based on the first input received by the UI unit 310. Based on the first TGC value, the control unit 320 automatically sets a TGC value corresponding to at least one second depth, except for the TGC value corresponding to the first depth, from among the plurality of TGC values.

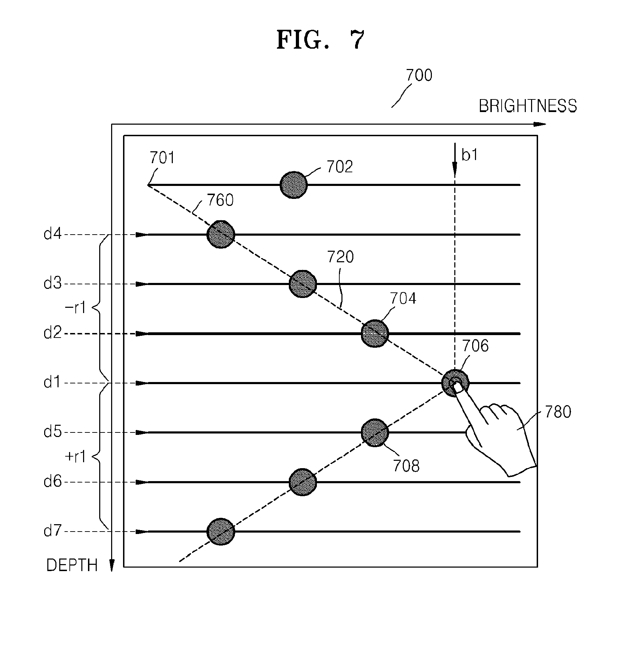

In detail, the control unit 320 may automatically set a plurality of TGC values based on an input received by the UI unit 310. For example, the control unit 320 may set a plurality of TGC values to form a straight line pattern, a curved line pattern, or a user-set pattern, as described in detail later with reference to FIGS. 7-18.

FIG. 4 is a block diagram of an ultrasound diagnosis apparatus 400 according to another embodiment of the present invention. The ultrasound diagnosis apparatus 400 has the same configuration as the ultrasound diagnosis apparatus 300 of FIG. 3 except that at least one of a memory 440 and a communication unit 450 is further included.

In detail, a UI unit 410, a control unit 420, and a display unit 430 of the ultrasound diagnosis apparatus 400 respectively correspond to the UI unit 310, the control unit 320, and the display unit 330 of the ultrasound diagnosis apparatus 300 of FIG. 3. Accordingly, descriptions about the ultrasound diagnosis apparatus 400 that are the same as those of the ultrasound diagnosis apparatus 300 of FIG. 3 are not repeated.

The memory 440 may store various types of data related to an ultrasound image. In detail, the memory 440 may store a captured ultrasound image. The memory 440 may also store information related to a plurality of TGC values. In detail, the memory 440 may store information related with a difference between the TGC values, a ratio therebetween, and conditions of the pattern so that the TGC values form a straight line pattern, a curved line pattern, or a user-set pattern. The memory 440 may store the TGC values such that the TGC values match with a plurality of depths.

The communication unit 450 may transmit or receive predetermined data to or from an external apparatus via a wired/wireless network. For example, when the ultrasound diagnosis apparatus 400 is the ultrasound diagnosis apparatus 100 of FIG. 1, the communication unit 450 corresponds to the communication unit 30 of FIG. 1 and may exchange data with the server 35, the medical apparatus 34, and the portable terminal 36.

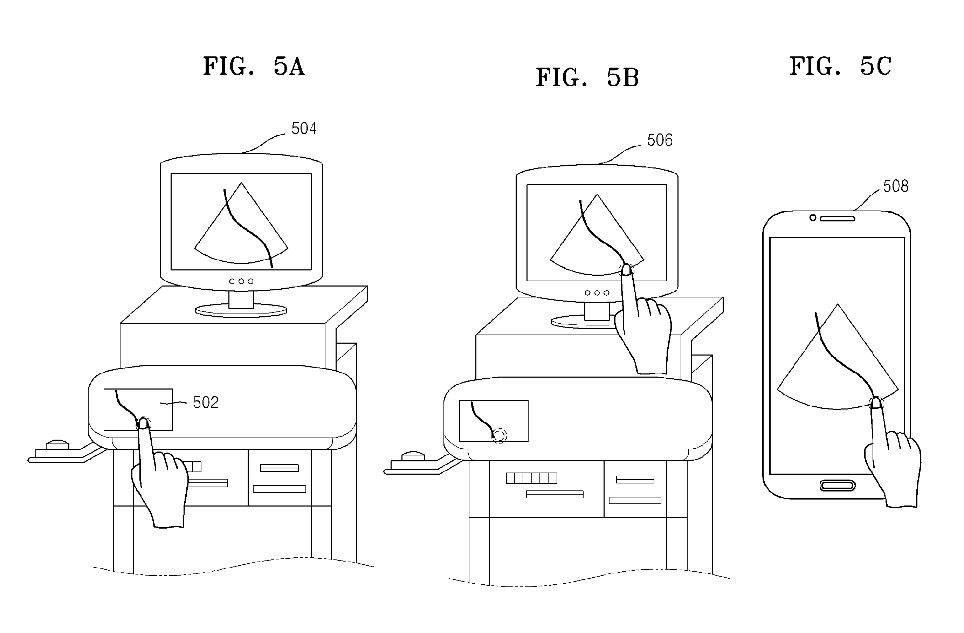

FIGS. 5A, 5B, and 5C illustrate embodiments of the present invention in which a user input is received.

As shown in FIG. 5A, the ultrasound diagnosis apparatus 300 may receive an input via a UI unit 502, which is provided in a control panel and separate from a display unit 504 that displays an ultrasound image.

Alternatively, as illustrated in FIG. 5B, the ultrasound diagnosis apparatus 300 may receive an input via a display unit 506. In other words, the UI unit 410 of FIG. 4, formed as a touch pad (not shown), may be coupled with a display panel (not shown) included in the display unit 430.

Alternatively, as illustrated in FIG. 5C, the ultrasound diagnosis apparatus 300 may be implemented by using a portable terminal. The portable terminal of FIG. 5C may correspond to the portable terminal 36 connected to the ultrasound diagnosis apparatus 100 of FIG. 1 via the network 3. For example, the ultrasound diagnosis apparatus 300 may be implemented by using any of various types of portable terminals, such as a PACS viewer, a smartphone, a laptop computer, and a tablet PC. A display unit 508 of the ultrasound diagnosis apparatus 300 of FIG. 5C may serve as a UI unit, similar to the embodiment of FIG. 5B. In other words, the display unit 508 of the ultrasound diagnosis apparatus 300 may be an input unit that senses a touch made by a user.

An embodiment in which the ultrasound diagnosis apparatus 300 receives a user input is not limited to the embodiments of FIGS. 5A, 5B, and 5C. In other words, the ultrasound diagnosis apparatus 300 may display an ultrasound image and receive an input, according to various methods.

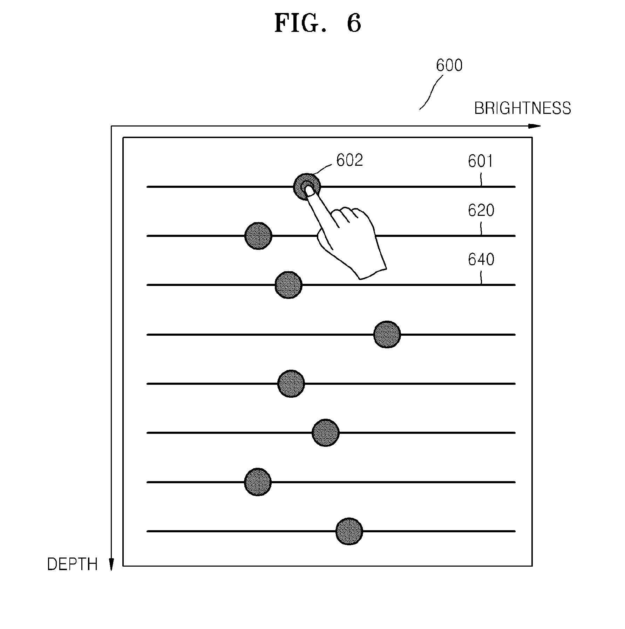

FIG. 6 is a view for describing a general operation of an ultrasound diagnosis apparatus to set TGC values. Referring to FIG. 6, a UI image 600 for setting a plurality of TGC values according to depths is illustrated. In the UI image 600, the horizontal axis indicates a gain value or a brightness value, which increases in a right direction. The vertical axis indicates a depth value corresponding to a depth at which a part of an object is located from the transducer 2200, where the depth value increases downwards. For example, a TGC value 601 corresponding to a first depth is used to compensate for a brightness of an ultrasound image corresponding to the first depth, and a TGC value 620 corresponding to a second depth is used to compensate for a brightness of the ultrasound image corresponding to the second depth. A TGC value 640 corresponding to a third depth is used to compensate for a brightness of the ultrasound image corresponding to the third depth. Depths of the locations of parts of the object from the transducer 2200 increase in the order of the first depth, the second depth, and the third depth, and compensation increases as a value as which a TGC value corresponding to each depth is set goes rightwards. In detail, as a TGC value corresponding to a predetermined depth increases, a gain value applied to an ultrasound echo signal acquired at the predetermined depth may increase, and thus the ultrasound echo signal acquired at the predetermined depth may be amplified more.

In the conventional art, independent inputs are needed to set a plurality of TGC values. In other words, even when a user sets the TGC value 601 corresponding to the first depth to be a first TGC value 602, a conventional ultrasound diagnosis apparatus needs other inputs in order to set TGC values other than the TGC value 601 corresponding to the first depth. For example, the conventional ultrasound diagnosis apparatus requires two independent inputs to set the TGC value 601 corresponding to the first depth as the first TGC value 602 and the TGC value 620 corresponding to the second depth as a second TGC value (not shown). Thus, a user is incovenienced in having to set TGC a number of times.

According to an embodiment of the present invention, a plurality of TGC values may be set based on a minimal number of user inputs. In detail, the UI unit 310 of the ultrasound diagnosis apparatus 300 receives a first input for setting a TGC value corresponding to a first depth from among a plurality of TGC values to be a first TGC value, via a UI image. The control unit 320 of the ultrasound diagnosis apparatus 300 sets the TGC value corresponding to the first depth as the first TGC value, based on the first input, and automatically sets a TGC value corresponding to at least one second depth, except for the TGC value corresponding to the first depth, from among the plurality of TGC values, based on the first TGC value.

In general, the magnitude of an ultrasound echo signal decreases as a transmission distance thereof increases. Thus, the control unit 320 may determine a TGC pattern so that more compensation is peformed for an object located more deeply and may automatically set a TGC value corresponding to at least one second depth, except for the TGC value corresponding to a first depth, according to the determined TGC pattern. Various TGC patterns may be set to set a TGC value corresponding to a second depth, based on the intention of a user and a part of a patient that is to be ultrasound-diagnosed. An ultrasound diagnosis apparatus according to an embodiment of the present invention may allow a user to easily set a plurality of TGC values according to depths so that the plurality of TGC values form a predetermined pattern.

TGC value setting operations of the ultrasound diagnosis apparatuses 300 and 400 will now be described in detail with reference to FIGS. 7-18.

FIG. 7 is a view for describing an operation of the ultrasound diagnosis apparatus 300 for setting TGC values, according to an embodiment of the present invention.

The display unit 330 of the ultrasound diagnosis apparatus 300 displays a UI image 700 of FIG. 7. The UI image 700 of FIG. 7 is the same as the UI image 600 of FIG. 6. Thus, a repeated description thereof will be omitted.

FIG. 7 illustrates a method in which the ultrasound diagnosis apparatus 300 automatically sets a plurality of TGC values. In detail, the UI unit 310 receives a first input 780 for setting a TGC value corresponding to a first depth to be a first TGC value 706. The control unit 320 sets the TGC value corresponding to the first depth to be the first TGC value 706, according to the first input 780, and automatically sets TGC values corresponding to a plurality of second depths except for the TGC value corresponding to the first depth. For example, when a user sets the TGC value 706 corresponding to a first depth d1 to be a value b1 via the UI unit 310, the control unit 320 may automatically set TGC values corresponding to second depths d2, d3, d4, d5, d6, and d7, except for the first depth d1.

The control unit 320 may only set TGC values corresponding to depths included in predetermined ranges starting from the first depth d1 (for example, ranges - r1 and +r1), based on user settings. For example, the control unit 320 may not automatically set a TGC value 702 not included in the predetermined ranges.

The control unit 320 may set TGC values corresponding to at least one second depth to be greater or smaller than the first TGC value 706 corresponding to the first depth d1 as the at least one second depth increases or decreases from the first depth d1.

In detail, the control unit 320 sets a TGC value 708 corresponding to the second depth d5 to be smaller than the first TGC value 706 as the second depth d5 is greater than the first depth d1. The control unit 320 sets a TGC value 704 corresponding to the second depth d2 to be smaller than the first TGC value 706 as the second depth d2 is less than the first depth d1. In other words, the control unit 320 may set TGC values corresponding to a plurality of second depths so that TGC values for different depths form a pattern 720 of FIG. 7.

The control unit 320 may automatically calculate the TGC values corresponding to the second depths. For example, as illustrated in FIG. 7, the control unit 320 may set the TGC values corresponding to the second depths, based on a ratio between the first depth d1 and the first TGC value 706. In detail, as the second depth d2 is less than the first depth d1, the control unit 320 sets the TGC value 704 having the second depth d2 to be on a straight line 760 that connects a starting point 701 and the first TGC value 706. When the second depth d5 is greater than the first depth d1, the control unit 320 may set the TGC value 708 corresponding to the second depth d5 to be smaller than the first TGC value 706 by a ratio of the straight line. Accordingly, the pattern 720 of FIG. 7 may be based on the straight line 760 of FIG. 7.

The control unit 320 may calculate the TGC values corresponding to the second depths according to user settings. For example, as a depth increases or decreases, a user may set increments or decrements of the TGC values corresponding to the second depths from the first TGC value 706 corresponding to the first depth d1. However, a method in which the control unit 320 sets the TGC values corresponding to the second depths is not limited to this method.

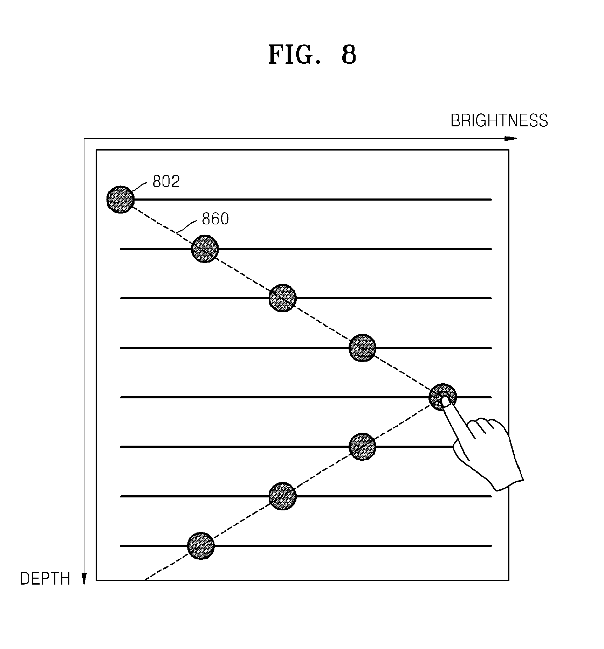

FIG. 8 is a view for describing an operation of the ultrasound diagnosis apparatus 300 to set TGC values, according to another embodiment of the present invention.

In contrast with FIG. 7, referring to FIG. 8, the control unit 320 sets all TGC values corresponding to a plurality of depths, based on a user's settings. For example, in contrast with the embodiment of FIG. 7, the control unit 320 automatically sets a TGC value 802 corresponding to a smallest depth that is far from a first depth and at which an object is located. In detail, the control unit 320 may set, as a starting point, the TGC value 802 corresponding to the smallest depth at which the object is located.

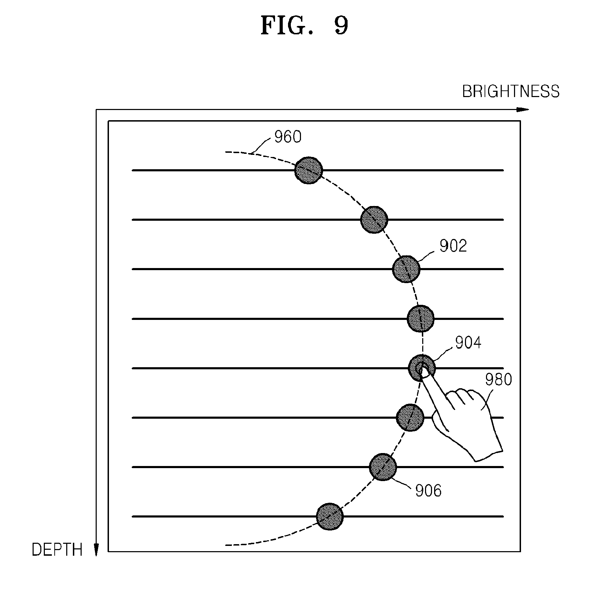

FIG. 9 is a view for describing an operation of the ultrasound diagnosis apparatus 300 to set TGC values, according to another embodiment of the present invention.

FIG. 9 illustrates a method in which the ultrasound diagnosis apparatus 300 sets a plurality of TGC values to form a curved line pattern 960. A first input 980 of FIG. 9 may be the same as the first input 780 of FIG. 7. Thus, a repeated description thereof will be omitted.

In detail, based on the first input 980, the control unit 320 automatically sets TGC values corresponding to a plurality of second depths, except for a TGC value corresponding to a first depth, to form the curved line pattern 960. For example, the control unit 320 may form the curved line pattern 960 based on a first TGC value 904 corresponding to the first depth d1 and may set a plurality of TGC values (e.g., TGC values 902 and 906) such that the plurality of TGC values are on the curved line pattern 960.

In particular, the curved line pattern 960 may easily be used to acquire an ultrasound image of a blood vessel. For example, when a user sets a TGC value of a central part of a blood vessel, the control unit 320 may automatically set a plurality of TGC values to form a curved line pattern based on the set TGC value of the central part of the blood vessel.

A curvature of the curved line pattern 960 may be set by a user or may be calculated by the control unit 320.

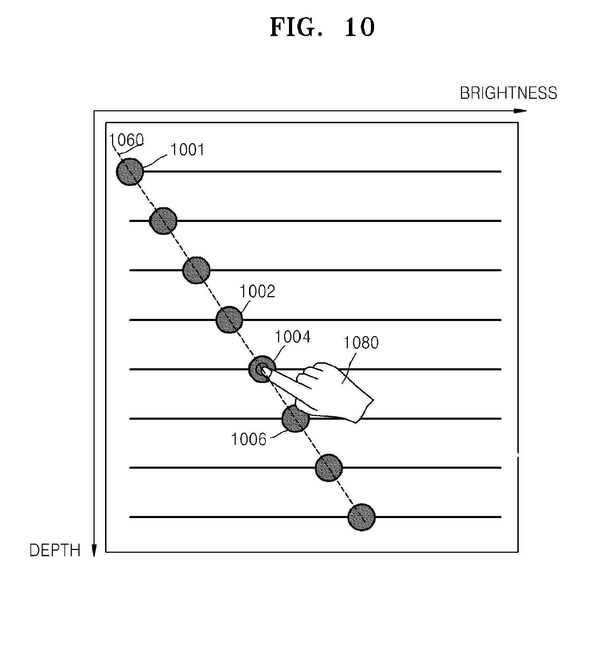

FIG. 10 is a view for describing an operation of the ultrasound diagnosis apparatus 300 to set TGC values, according to another embodiment of the present invention.

FIG. 10 illustrates a method in which the ultrasound diagnosis apparatus 300 sets a plurality of TGC values to form a straight line pattern 1060. A first input 1080 of FIG. 10 may be the same as the first input 780 of FIG. 7. Thus, a repeated description thereof will be omitted.

In detail, based on the first input 1080, the control unit 320 automatically sets TGC values corresponding to a plurality of second depths, except for a TGC value corresponding to a first depth, to form the straight line pattern 1060. For example, the control unit 320 may form the straight line pattern 1060 based on a first TGC value 1004 corresponding to the first depth d1 and may set a plurality of TGC values (e.g., TGC values 1002 and 1006) to be in the straight line pattern 1060.

The straight line pattern 1060 may be used in a general TGC setting method. As described above, in TGC, if the speeds of ultrasound echo signals reflected from different parts of an object are constant and the reflection coefficients of the object are constant, more compensation with respect to an ultrasound echo signal needs to be done as a part of the object is located deeper. For example, when a user sets a TGC value corresponding to a predetermined depth at which an object is located, the control unit 320 may automatically set a plurality of TGC values such that more compensation is performed as an object is located deeper, based on the straight line pattern 1060 and the set TGC value corresponding to the predetermined depth.

An inclination of the straight line pattern 1060 may be set by a user or may be calculated by the control unit 320.

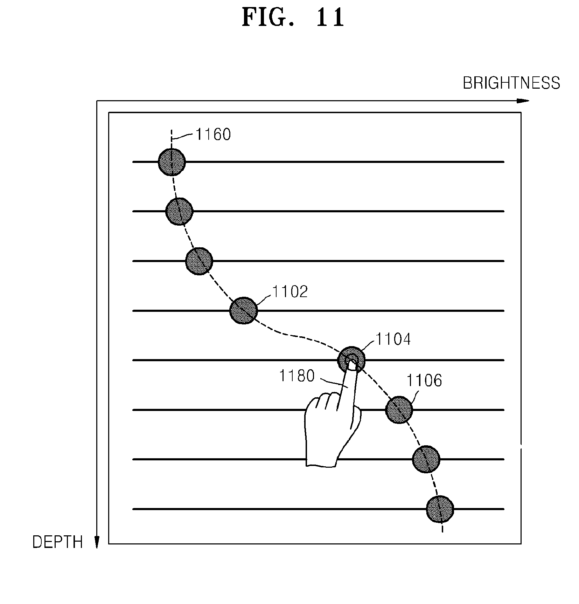

FIG. 11 is a view for describing an operation of the ultrasound diagnosis apparatus 300 to set TGC values, according to another embodiment of the present invention.

FIG. 11 illustrates a method in which the ultrasound diagnosis apparatus 300 sets a plurality of TGC values in a user-set pattern 1160. A first input 1180 of FIG. 11 may be the same as the first input 780 of FIG. 7. Thus, a repeated description thereof will be omitted.

In detail, based on the first input 1180, the control unit 320 automatically sets TGC values corresponding to a plurality of second depths, except for a TGC value corresponding to a first depth, to form the user-set pattern 1160. For example, the control unit 320 may form the user-set pattern 1160 based on a first TGC value 1104 corresponding to the first depth d1 and may set a plurality of TGC values (e.g., TGC values 1102 and 1106) such that the plurality of TGC values are on the user-set pattern 1160.

The user-set pattern 1060 may have various forms. A user may set suitable patterns according to ultrasound diagnosis environments.

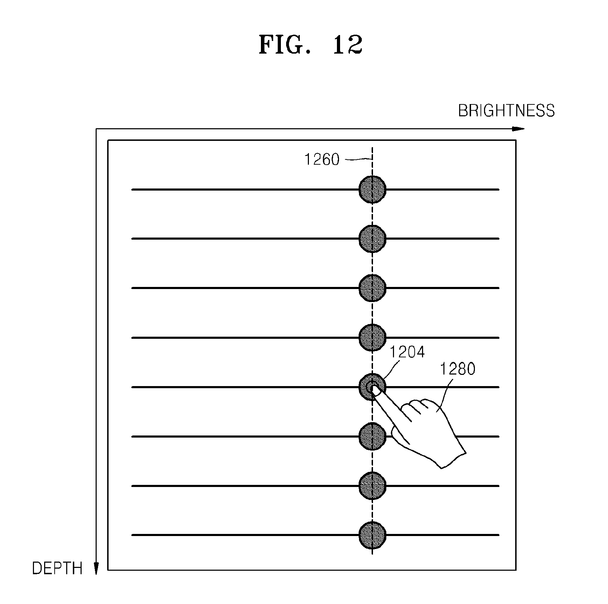

FIG. 12 is a view for describing an operation of the ultrasound diagnosis apparatus 300 to set TGC values, according to another embodiment of the present invention.

FIG. 12 illustrates a method in which the ultrasound diagnosis apparatus 300 sets a plurality of TGC values to be an identical value. A first input 1280 of FIG. 12 may be the same as the first input 780 of FIG. 7. Thus, a repeated description thereof will be omitted.

In detail, the control unit 320 automatically sets TGC values corresponding to a plurality of second depths, except for a TGC value corresponding to a first depth, to be a first TGC value 1204, according to the first input 1280.

The ultrasound diagnosis apparatus 300 may set a plurality of TGC values according to a plurality of user inputs. A user may more efficiently set a plurality of TGC values by using a plurality of user inputs. The ultrasound diagnosis apparatus 300 setting a plurality of TGC values according to a plurality of user inputs will now be described.

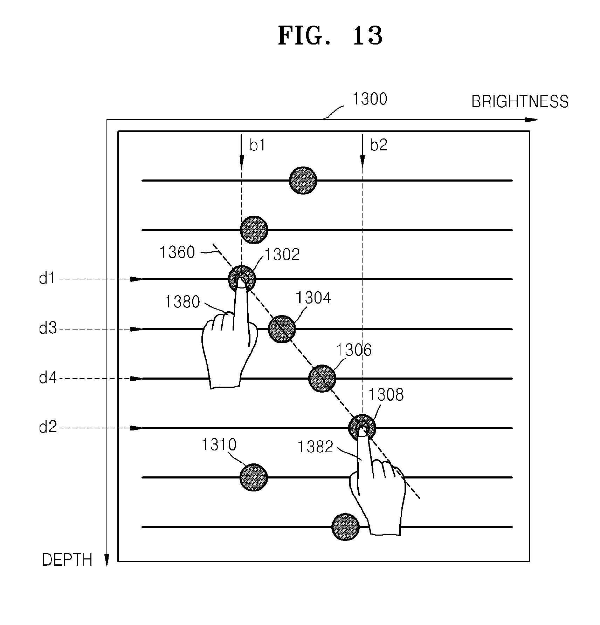

FIG. 13 is a view for describing an operation of the ultrasound diagnosis apparatus 300 to set TGC values, according to another embodiment of the present invention.

The display unit 330 of the ultrasound diagnosis apparatus 300 displays a UI image 1300 of FIG. 13. The UI image 1300 of FIG. 13 corresponds to the UI image 600 of FIG. 6. Thus, a repeated description thereof will be omitted.

FIG. 13 illustrates a method in which the ultrasound diagnosis apparatus 300 sets a plurality of TGC values 1304 and 1306 according to a first input 1380 and a second input 1382, respectively, to form a straight line pattern 1360. In detail, the UI unit 310 receives the first input 1380 and the second input 1382 to respectively set a TGC value corresponding to a first depth d1 and a TGC value corresponding to a second depth d2 from among a plurality of TGC values to be respectively a first TGC value 1302 (i.e., b1) and a second TGC value 1308 (i.e., b2). The UI unit 310 may simultaneously or sequentially receive the first input 1380 and the second input 1382.

The control unit 320 sets, based on the first input 1380 and the second input 1382, the TGC values corresponding to the first and second depths d1 and d2 to be the first TGC value 1302 and the second TGC value 1308, respectively, and automatically sets, based on the first and second TGC values 1302 and 1308, a TGC value corresponding to at least one third depth (for example, third depths d3 and d4), except for the TGC values corresponding to the first and second depth d1 and d2.

According to an embodiment of the present invention, the at least one third depth may be a depth between the first and the second depths d1 and d2, based on a user's settings. In detail, the control unit 320 may automatically set TGC values 1304 and 1306 corresponding to the third depths d3 and d4 between the first and second depths d1 and d2 and may not set a TGC value corresponding to a depth not present between the first and second depths d1 and d2 (for example, a TGC value 1310). However, the third depths are not limited to this embodiment.

The control unit 320 may generate a straight line pattern 1360 connecting the first TGC value 1302 and the second TGC value 1308 to each other and may automatically set the TGC values 1304 and 1306 corresponding to the third depths d3 and d4 in the straight line pattern 1360. Although the TGC values between the first TGC value 1302 and the second TGC value 1308 are in the straight line pattern 1360 in FIG. 13, the TGC values may be present in any of various patterns as illustrated in FIGS. 8-12.

A user may easily and efficiently set the straight line pattern 1360 and the third depths, based on the first input 1380 and the second input 1382.

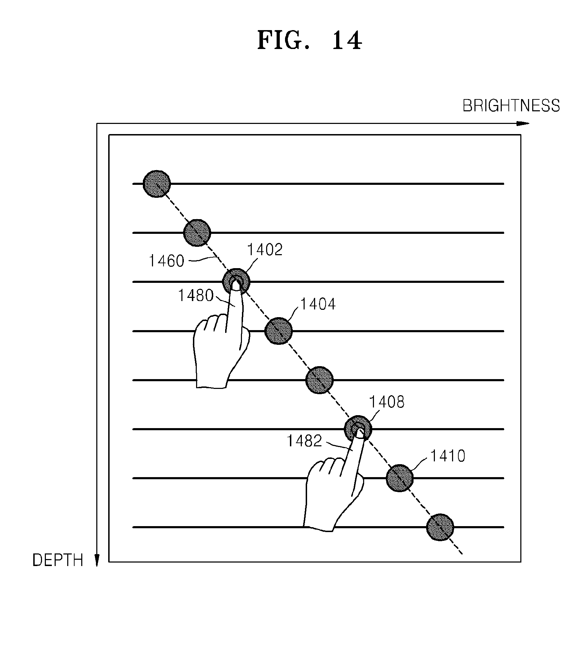

FIG. 14 is a view for describing an operation of the ultrasound diagnosis apparatus 300 to set TGC values, according to another embodiment of the present invention.

In contrast with FIG. 13, referring to FIG. 14, the control unit 320 sets all TGC values corresponding to a plurality of depths, based on a user's settings. In FIG. 14, third depths include all depths but first and second depths. Accordingly, the control unit 320 may generate a straight line pattern 1460 connecting a first TGC value 1402 corresponding to the first depth and a second TGC value 1408 corresponding to the second depth to each other and may automatically set TGC values 1404 and 1410 corresponding to third depths in the straight line pattern 1460.

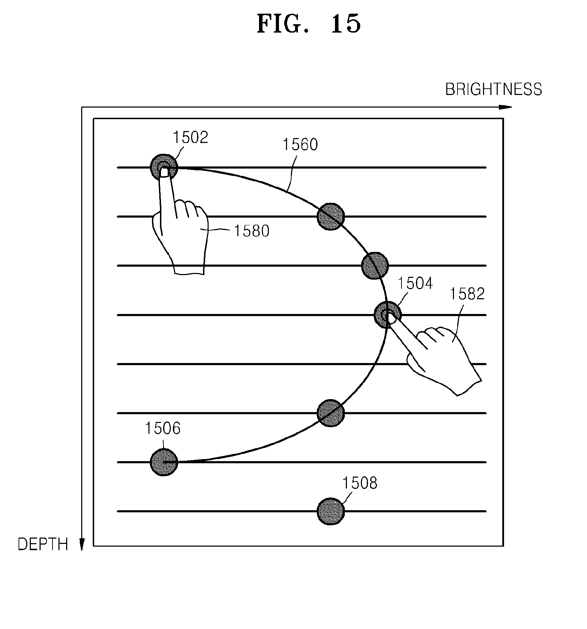

FIG. 15 is a view for describing an operation of the ultrasound diagnosis apparatus 300 to set TGC values, according to another embodiment of the present invention.

FIG. 15 illustrates a method in which the ultrasound diagnosis apparatus 300 sets a plurality of TGC values to (form a curved line pattern 1560. A first input 1580 and a second input 1582 of FIG. 15 may be the same as the first input 1380 and the second input 1382 of FIG. 13, respectively. Thus, a repeated description thereof will be omitted.

The control unit 320 of FIG. 15 may generate a curved line pattern 1560 that connects a first TGC value corresponding to a first depth and a second TGC value corresponding to a second depth.

According to an embodiment of the present invention, a third depth may be set to be a depth included in the curved line pattern 1560, based on a user's settings. For example, in FIG. 15, when the curved line pattern 1560 is generated based on the first input 1580 and the second input 1582, the third depth may be set to be at least one depth between a start 1502 and an end 1506 of the curved line pattern 1560. However, the third depth is not limited to this embodiment.

The control unit 320 may automatically set TGC values including the end 1506 corresponding to third depths to be present on the curved line pattern 1560 and may not set a TGC value 1508 corresponding to a depth not included in the third depths.

A user may easily and efficiently set the curved line pattern 1560 and the third depths, based on the first input 1580 and the second input 1582.

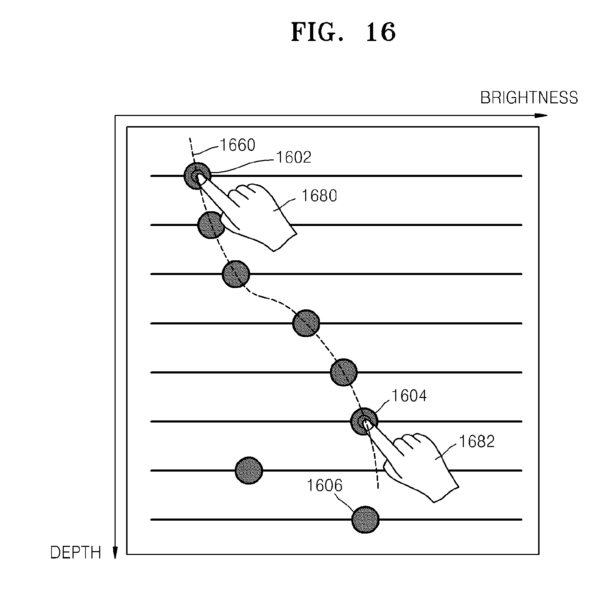

FIG. 16 is a view for describing an operation of the ultrasound diagnosis apparatus 300 to set TGC values, according to another embodiment of the present invention.

FIG. 16 illustrates a method in which the ultrasound diagnosis apparatus 300 sets a plurality of TGC values to form a user-set pattern 1660. A first input 1680 and a second input 1682 of FIG. 16 may be the same as the first input 1380 and the second input 1382 of FIG. 13, respectively. Thus, a repeated description thereof will be omitted.

According to an embodiment of the present invention, third depths may be depths between a first depth and a second depth, based on a user's settings. The control unit 320 may automatically set TGC values corresponding to the third depths and may not set a TGC value 1606 corresponding to a depth not included in the third depths. However, the third depths are not limited to this embodiment.

The control unit 320 may generate the user-set pattern 1660, connecting a first TGC value 1602 corresponding to the first depth and a second TGC value 1604 corresponding to the second depth and may automatically set the TGC values corresponding to the third depths to be on the user-set pattern 1660.

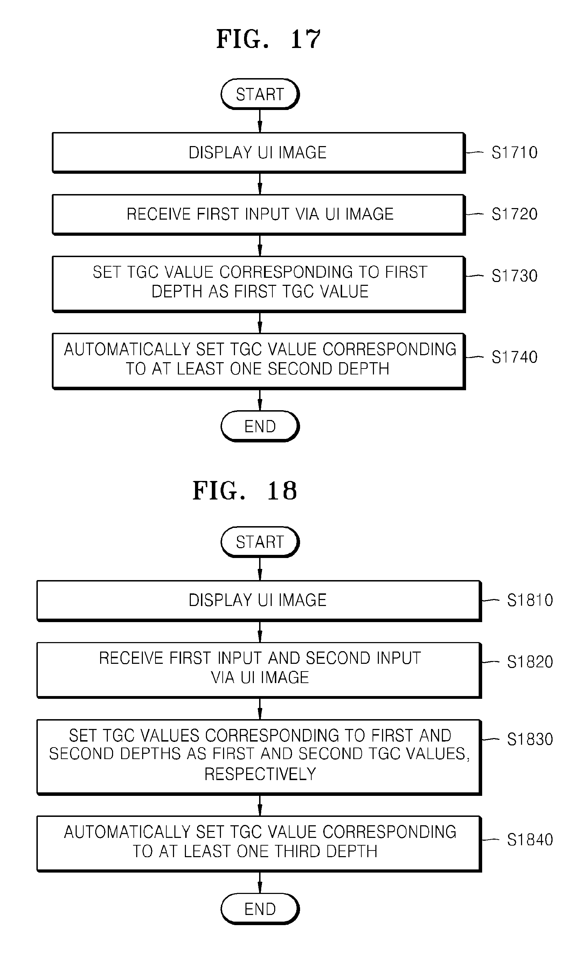

FIG. 17 is a flowchart of a method of setting TGC values, the method performed by an ultrasound diagnosis apparatus, according to an embodiment of the present invention. The method of setting TGC values may be performed by the ultrasound diagnosis apparatuses 300 and 400 described above with reference to FIGS. 3 and 4. Operations of the TGC value setting method include the same technical spirits as the above-described operations of the ultrasound diagnosis apparatuses 300 and 400. A repeated description provided above with reference to FIGS. 1 through 16 is omitted herein.

In operation S1710, the display unit 330 of the ultrasound diagnosis apparatus 300 may display a UI image for setting a plurality of TGC values according to depths, under the control of the control unit 320.

In operation S1720, the UI unit 310 of the ultrasound diagnosis apparatus 300 may receive a first input for setting a TGC value corresponding to a first depth from among the plurality of TGC values to be a first TGC value, via the UI image.

In operations S1730 and S1740, the control unit 320 of the ultrasound diagnosis apparatus 300 may set the TGC value corresponding to the first depth as the first TGC value, based on the first input, and automatically set a TGC value corresponding to at least one second depth except for the TGC value corresponding to the first depth from among the plurality of TGC values, based on the first TGC value. For example, the plurality of TGC values may be set as described above with reference to FIGS. 7-12.

FIG. 18 is a flowchart of a method of setting TGC values, the method performed by an ultrasound diagnosis apparatus, according to another embodiment of the present invention. The TGC value setting method may be performed by the ultrasound diagnosis apparatuses 300 and 400 described above with reference to FIGS. 3 and 4. Operations of the TGC value setting method include the same technical spirits as the above-described operations of the ultrasound diagnosis apparatuses 300 and 400. A repeated description provided above with reference to FIGS. 1 through 16 is omitted herein.

In operation S1810, the display unit 330 of the ultrasound diagnosis apparatus 300 may display a UI image for setting a plurality of TGC values according to depths, under the control of the control unit 320.

In operation S1820, the UI unit 310 of the ultrasound diagnosis apparatus 300 may receive a first input and a second input for respectively setting a TGC value corresponding to a first depth and a TGC value corresponding to a second depth from among the plurality of TGC values to be first and second TGC values, respectively, via the UI image.

In operations S1830 and S1840, the control unit 320 of the ultrasound diagnosis apparatus 300 may set the TGC values corresponding to the first and second depths as the first and second TGC values, respectively, based on the first and second inputs and automatically set a TGC value corresponding to at least one third depth, except for the TGC values corresponding to the first and second depths, based on the first and second TGC values. For example, the plurality of TGC values may be set as described above with reference to FIGS. 13-16.

As described above, in an ultrasound diagnosis apparatus and a TGC setting method performed by the ultrasound diagnosis apparatus, according to the one or more of the above embodiments of the present invention, TGC of an ultrasound image is automatically set based on a minimal number of user inputs. Accordingly, user convenience may increase.

The embodiments of the present invention can be written as computer programs and can be implemented in general-use digital computers that execute the programs using a computer-readable recording medium.

Examples of the computer-readable recording medium include magnetic storage media (e.g., ROM, floppy disks, hard disks, etc.), optical recording media (e.g., CD-ROMs, or DVDs), etc.

The exemplary embodiments should be considered in descriptive sense only and not for purposes of limitation. Descriptions of features or aspects within each embodiment should typically be considered as available for other similar features or aspects in other embodiments.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. An ultrasound diagnosis apparatus comprising:
a display unit which displays a user interface (UI) image for setting a plurality of time gain compensation (TGC) values according to depths;
a UI unit which receives a first input for setting a TGC value corresponding to a first depth from among the plurality of TGC values to be a first TGC value, via the UI image; and
a control unit which sets the TGC value corresponding to the first depth to be the first TGC value, based on the first input, and automatically sets a TGC value corresponding to at least one second depth except for the TGC value corresponding to the first depth from among the plurality of TGC values, based on the first TGC value.

2. The ultrasound diagnosis apparatus of claim 1, wherein the control unit sets the TGC value corresponding to the at least one second depth to be greater or smaller than the first TGC value corresponding to the first depth as the at least one second depth increases or decreases from the first depth.

3. The ultrasound diagnosis apparatus of claim 2, wherein the control unit sets the TGC value corresponding to the at least one second depth to be smaller than the first TGC value corresponding to the first depth when the at least one second depth decreases from the first depth.

4. The ultrasound diagnosis apparatus of claim 3, wherein the control unit sets the TGC value corresponding to the at least one second depth to be smaller than the first TGC value corresponding to the first depth when the at least one second depth increases from the first depth.

5. The ultrasound diagnosis apparatus of claim 3, wherein the control unit sets the TGC value corresponding to the at least one second depth to be greater than the first TGC value corresponding to the first depth when the at least one second depth increases from the first depth.

6. The ultrasound diagnosis apparatus of claim 1, wherein the control unit sets the TGC value corresponding to the at least one second depth to form a straight line pattern together with the first TGC value corresponding to the first depth .

7. The ultrasound diagnosis apparatus of claim 1, wherein the control unit sets the TGC value corresponding to the at least one second depth to form a curved line pattern together with the first TGC value corresponding to the first depth.

8. The ultrasound diagnosis apparatus of claim 1, wherein the control unit sets the TGC value corresponding to the at least one second depth to form a user-set pattern together with the first TGC value corresponding to the first depth.

9. The ultrasound diagnosis apparatus of claim 1, wherein the control unit sets the TGC value corresponding to the at least one second depth to be the same as the first TGC value.

10. The ultrasound diagnosis apparatus of claim 1, wherein the control unit sets the TGC value corresponding to the at least one second depth, based on a part of an object that is to be ultrasound-diagnosed.

11. The ultrasound diagnosis apparatus of claim 1, wherein the UI unit which receives a first input and a second input for respectively setting a TGC value corresponding to a first depth and a TGC value corresponding to a second depth from among the plurality of TGC values to be first and second TGC values, respectively, via the UI image; and
the control unit which sets the TGC values corresponding to the first and second depths as the first and second TGC values, respectively, based on the first and second inputs, and automatically sets a TGC value corresponding to at least one third depth, except for the TGC values corresponding to the first and second depths, based on the first and second TGC values.

12. A time gain compensation (TGC) setting method performed by an ultrasound diagnosis apparatus, the method comprising:
displaying a user interface (UI) image for setting a plurality of TGC values according to depths;
receiving a first input for setting a TGC value corresponding to a first depth from among the plurality of TGC values to be a first TGC value, via the UI image; and
setting the TGC value corresponding to the first depth to be the first TGC value, based on the first input and automatically setting a TGC value corresponding to at least one second depth, except for the TGC value corresponding to the first depth, from among the plurality of TGC values, based on the first TGC value.

13. The TGC setting method of claim 12, wherein the automatically setting of the TGC value corresponding to the at least one second depth comprises setting the TGC value corresponding to the at least one second depth to be greater or smaller than the first TGC value corresponding to the first depth as the at least one second depth increases or decreases from the first depth.

14. The TGC setting method of claim 12, wherein the automatically setting of the TGC value corresponding to the at least one second depth comprises setting the TGC value corresponding to the at least one second depth to form a straight line pattern together with the first TGC value corresponding to the first depth.

15. The TGC setting method of claim 12, wherein the automatically setting of the TGC value corresponding to the at least one second depth comprises setting the TGC value corresponding to the at least one second depth, based on a part of an object that is to be ultrasound-diagnosed.
